# EUROPEAN PATENT SPECIFICATION

(11) **EP 1 572 152 B1**
(45) Date of publication and mention of the grant of the patent: **13.02.2008**
(21) Application number: 03814246.9
(22) Date of filing: 19.12.2003
(51) Int. Cl.: A61K 9/127, A61K 31/196

(54) **LIPOSOMAL ANALGESIC FORMULATION COMPRISING DICLOFENAC AND ITS USE FOR TREATMENT OF HORSES**
DICOLFENAC-HALTIGE LIPOSOMALE SCHMERZSTILLENDE FORMULIERUNG UND IHRE VERWENDUNG ZUR BEHANDLUNG VON PFERDEN
FORMULATION ANALGESIQUE LIPOSOMIQUE COMPRENANT DU DICLOFENAC ET SON UTILISATION POUR LE TRAITEMENT DES CHEVAUX

(30) Priority: 20.12.2002 US 327575; 25.11.2003 US 721373
(43) Date of publication of application: 14.09.2005
(73) Proprietor: IDEXX LABORATORIES, INC., Westbrook, ME 04092 (US)
(72) Inventor: HEPLER, Douglas, I., McLeansville, Nc 27301 (US); LYNN, Randy, Carl, Greensboro, NC 27358 (US)
(74) Representative: Savic, Bojan
(86) International application number: PCT/US2003/040717
(87) International publication number: WO 2004/057950

(56) References cited:
- WO-A-95/35095
- DE-A1- 19 940 227
- US-A- 5 738 869
- US-A- 6 117 455
- US-B2- 6 287 587
- DATABASE BIOSIS [Online] BIOSCIENCES INFORMATION SERVICE, PHILADELPHIA, PA, US; December 1999 (1999-12), GRACE DAVID ET AL: "Topical diclofenac versus placebo: A double blind, randomized clinical trial in patients with osteoarthritis of the knee" XP002358996 Database accession no. PREV200000054655 & JOURNAL OF RHEUMATOLOGY, vol. 26, no. 12, December 1999 (1999-12), pages 2659-2663, ISSN: 0315-162X
- CURT HUNNIUS, ARTUR BURGER, HELMUT WACHTER: "Hunnius - Pharmazeutisches Wörterbuch" 1998, DE GRUYTER , BERLIN, NEW YORK , XP002358997 * page 833 *

## Description

### FIELD OF THE INVENTION

This invention is in the field of liposomal non-steroidal anti-inflammatory formulations.

### SUMMARY OF THE INVENTION

One embodiment of the invention provides a method for treating lameness, navicular syndrome, osteoarthritis or a combination thereof in a horse. The method comprises topically administering to the a horse a formulation comprising: about 0.1% to about 5% diclofenac and about 0.5% to about 20% phospholipids, whereby lameness, navicular syndrome, osteoarthritis or a combination thereof in the horse is treated. The formulation can further comprise about 0.1% to about 10% vitamin E. The formulation can further comprise about 1% to about 20% alkylane glycol, and about 1% to about 50% (C₁-C₆) alcohol. The formulation can comprise about 1% diclofenac salt, about 5% propylene glycol, about 6% ethanol, about 1% vitamin E acetate, about 10% phospholipid, and about 77% water. The formulation can comprise about 2 to about 10 micron liposomes. The formulation can be topically administered twice daily. About 20 mg to about 120 mg of diclofenac can be applied as a single dose to the horse. About 70 mg to about 80 mg of diclofenac can be applied as a single dose to the horse. Two doses per day can be applied to the horse for at least about three days. The vitamin E can be a C₂-C₆ ester of vitamin E. The vitamin E can be vitamin E acetate. The diclofenac can be a diclofenac salt. The alkylane glycol can be propylene glycol. The C₁-C₆ alcohol can be ethanol.

### DETAILED DESCRIPTION OF THE INVENTION

It has been discovered that a liposome formulation containing about 0.1% to about 5% diclofenac is an effective topical anti-inflammatory treatment for lameness, navicular syndrome, osteoarthritis, or a combination thereof in horses. More particularly it has been discovered that a formulation containing, for example, vitamin E, phospholipid and diclofenac salt such as the sodium or potassium salt is a highly effective topical anti-inflammatory formulation that is particularly effective in treating lameness in horses.

### Compositions

A formulation refers to a composition in a form suitable for topical administration to a subject. A subject can be a member of the *Equidae* family, for example, a horse.

A formulation of the invention comprises about 0.1% to about 5% diclofenac. All percentages are based on total weight of the formulation. Diclofenac is described in U.S. Pat. No. 3,558,690 and in Merck Index 3071 eleventh Edition. Various diclofenac products are described in Physicians Desk Reference. In one embodiment, a formulation of the invention comprises about 0.7 to about 1.3% diclofenac, and in another embodiment, a formulation comprises about 1% diclofenac. Diclofenac can be a salt of diclofenac such as diclofenac sodium salt, diclofenac potassium salt, diclofenac diethylammonium salt, diclofenac hydroxyethylpyrrolidine salt, diclofenac diethylamine salt, diclofenac rubidium salt, diclofenac caesium salt, or diclofenac calcium salt.

A formulation of the invention can comprise about 0.1% to about 10% vitamin E. In one embodiment of the invention, vitamin E comprises about 0.5 to about 1.5% of a formulation, in another embodiment of the invention the formulation comprises about 1% vitamin E. Vitamin E can be a C₂ - C₆ ester of vitamin E, for example, vitamin E acetate.

A formulation of the invention can comprise about 0.5 to about 20% phospholipids. In another embodiment of the invention, the formulation can comprise about 5% to about 15% phospholipids. In one embodiment of the invention, a formulation can comprise about 10% phospholipids. Any phospholipid suitable for topical administration can be used in a formulation of the invention. See e.g., Gennaro, Remington: The Science and Practice of Pharmacy, (June 2003), Lippincott Williams & Wilkins Publishers, Philadelphia, PA.; Ansel et al., Pharmaceutical Dosage Forms and Drug Delivery Systems, 7th edition (July 2004), Lippincott Williams & Wilkins Publishers, Philadelphia, PA; U.S. Patent 4,937,078.

In one embodiment of the invention a phospholipid is phospholipon 90H HYD Lechthin (Aventis, Bridgewater Crossings, NJ, USA). Other useful lipids are obtainable from a number of sources. Natural phosphatide mixtures from egg or soy containing more than 70% phosphatidylcholine are obtained from a number of commercial sources such as Sigma Chemical of St. Louis, MO, and Lipoid KG, Ludwigshafen, West Ger., Hepar of Franklin, Ohio. Hepar supplies egg phosphatidylcholine. Other sources of lipid such as soy phosphatidylcholine are American Lecithin, Woodside, L.I., NY, and Riceland Foods, Little Rock, Ark. Phosphatidic acid of 99% purity is obtained from Avanti Chemical of Birmingham, AL. Purified natural soybean lecithin having about 80% phosphotidylcholine 2% lysophosphatidylcholine, 4% phosphatic acid and about 1% monophosphatidyllinosilol are also suitable for practicing the invention. Those skilled in the art will recognize a variety of suitable phospholipid compositions useful in the present invention. For example, U.S. Patent 5,154,930 describes the use of liposomes in the delivery of diclofenac. U.S. Patent 5,738,869 describes a phospholipid transdermal drug delivery system containing □-tocopherol, aliphatic alcohol and diclofenac. U.S. Patent 4,761,288 discloses the use of multilamellar lipid vesicles having a saturated solution and solid form of a drug captured therein. U.S. Patent 4,897,269 relates to topical administration of drugs using drugs captured in lipid vesicles. U.S. Patent 6,423,338 describes phospholipids containing microcrystals of drugs.

In one embodiment of the invention, the phospholipids form liposomes or vesicles of, for example, about 1 to about 30 microns, more preferably from about 2 to about 10 microns

A formulation of the invention can comprise about 1% to about 20% alkylane glycol, in another embodiment a formulation can comprise about 3% to about 7% alkylane glycol, and in another embodiment of the invention a formulation can comprise about 5% alkylane glycol. Any alkylane glycol suitable for topical administration can be used in the invention. See e.g., Gennaro, Remington: The Science and Practice of Pharmacy; Ansel et al., Pharmaceutical Dosage Forms and Drug Delivery Systems. In one embodiment of the invention an alkylane glycol is propylene glycol.

A formulation of the invention can comprise about 1% to about 50% (C₁-C₆) alcohol, in another embodiment a formulation can comprise about 3% to about 9% (C₁-C₆) alcohol, and in another embodiment of the invention a formulation can comprise about 6% (C₁-C₆) alcohol. Any alkylane glycol suitable for topical administration can be used in the invention. See e.g., Gennaro, Remington: The Science and Practice of Pharmacy; Ansel et al., Pharmaceutical Dosage Forms and Drug Delivery Systems. In one embodiment of the invention an alkylane glycol is propylene glycol.

Formulations of the invention can comprise about 99% to about 30% water, excipients, or other minor ingredients (i.e. preservatives, perfumes, colorants and the like). In one embodiment of the invention, a formulation comprises about 90%, about 80%, about 77%, about 70%, about 60%, about 50%, about 40%, or about 30% water. Excipients and minor ingredients suitable for the formulations of the invention are described in, for example, Gennaro, Remington: The Science and Practice of Pharmacy; Ansel et al., Pharmaceutical Dosage Forms and Drug Delivery Systems.

One embodiment of the invention provides a formulation that comprises about 1% vitamin E acetate, about 10% phospholipid, about 1% diclofenac, about 5% propylene glycol, about 6% ethanol and about 77% water. The formulation can comprise about 2 to about 10 micron lipid vesicles or liposomes that contain diclofenac. The composition can also contain small amounts of benzethonium chloride or other preservative.

One embodiment of the invention provides a formulation having about 0.5% to about 1.5% of Vitamin E ester, about 5% to about 15% of phospholipid, about 0.7% to about 1.3% diclofenac, about 3% to about 7% alkylane glycol, about 3% to about 9% of (C₁-C₆) alcohol and the remainder water or other minor ingredients (i.e. preservatives, perfumes, colorants and the like).

One embodiment of the invention provides a formulation comprising about 0.5% to about 1.5% of vitamin E, or a C₂ - C₆ ester of vitamin E; about 5% to about15% phospholipid; about 3 to about 7% alkylane glycol comprising about 2-5 carbon atoms, preferably propylene glycol; about 3% to about 9% of (C₁ - C₆) alcohol, preferably ethanol; about 0.7% to about 1.3% diclofenac, preferably about 1%; and the remainder water, wherein the formulation contains about 2 to about 10 micron lipid vesicles.

### Methods of Treatment

Formulations of the invention can be used to treat members of the *Equidae* family, including, for example, horses. Treatment means the elimination or reduction of one or more symptoms of lameness, osteoarthritis, navicular syndrome, or combinations thereof.

Lameness is an irregular gait caused by perception of a pain by partially or fully bearing weight on one or more legs. Lameness can be intermittent or continuous for a period of days, weeks or months.

Lameness can result from, for example, painful lesions on bone structure, cartilage, ligaments, the synovial membrane or connective tissue; from a change in bone architecture and/or in the bone growth cartilages at the site of lameness, such as, losses of bone substance, the formation of cysts, deformation of the bone or excessive thickening of the growth cartilages; from a change in the structure of the articular cartilages, such as, for example, erosions of the cartilaginous surfaces and/or a change in the synovial membrane and/or a change in the articular ligaments; from an anomaly of local vascularization, for example, a reduction in vascularization; from a change in muscle development, such as muscular atrophy, or a combination thereof.

Navicular syndrome is one of several conditions leading to heel soreness or lameness. Navicular syndrome is usually a chronic bilateral foreleg lameness. Horses with navicular syndrome can have a choppy, shuffling type gait and can wear the toes of their feet leaving the heels to grow longer. Horses with an upright conformation, small feet, or that are improperly shod can be at higher risk for the syndrome since they transmit more concussive force through the navicular region. The more pressure that is applied to the navicular bone from the deep flexor tendon, the more likely the horse will suffer from navicular disease. This repetitive force can result in damage and inflammation to the navicular bone resulting in navicular syndrome. Diagnosis of navicular syndrome is based on a clinical examination (characteristic gait, hoof tester response, specific nerve blocks, and palpation) and radiographic evaluation.

Osteoarthritis is a degenerative joint disease. The condition is characterized by degeneration of the articular cartilage, hypertrophy of bone at the margins and changes in the synovial membrane. Osteoarthritis primarily affects weight-bearing joints in horses such as the hocks (ankles) where it can be referred to as bone spavins, fetlocks, pastern joints and coffin joints, where it is referred to as ringbone when more severe, knee joints, spine (neck and back). Osteoarthritis can affect other joints especially where previous injury has occurred or areas where there has been repetitive or abnormal stress. Osteoarthritis can result from injury, loose joints, an abnormal growth pattern, or inherited factors.

### Administration

A formulation of the invention is topically administered to a subject. Topical administration means directly layering, applying or spreading upon epidermal tissue, especially outer skin or membrane, including the hoof. A formulation is applied to or near the affected area of the subject, for example to the area of a joint affected by lameness. The formulation can be administered to any affected area of the subject.

A three to seven inch ribbon, preferably about a five inch ribbon, of a formulation is generally applied once or twice daily for about 3 to about 10 days. This equates to between about 20 mg to about 120 mg diclofenac, preferably between about 70 mg to about 80 mg per dose (i.e., per application). Other dosages can be used, such as those disclosed in US Patent No. 4,937,078. A formulation can also be administered as one, two, three or more doses per day. Administration can continue for anywhere between 1 and 30 days or longer.

The following examples are intended to illustrate but not limit the invention. While they are typical of those that might be used, other procedures known to those of ordinary skill in the art may alternatively be used.

All patents and publications mentioned in the specification are indicative of the levels of skill of those skilled in the art to which the invention pertains. All references cited in this disclosure are incorporated by reference to the same extent as if each reference had been incorporated by reference in its entirety individually.

The methods and compositions described herein as presently representative of preferred embodiments are exemplary and are not intended as limitations on the scope of the invention. Changes therein and other uses will occur to those skilled in the art, which are encompassed within the spirit of the invention, are defined by the scope of the claims.

The invention illustratively described herein suitably can be practiced in the absence of any element or elements, limitation or limitations that are not specifically disclosed herein. Thus, for example, in each instance herein any of the terms "comprising", "consisting essentially of, and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments, optional features, modification and variation of the concepts herein disclosed may be resorted to by those skilled in the art, and that such modifications and variations are considered to be within the scope of this invention as defined by the description and the appended claims.

In addition, where features or aspects of the invention are described in terms of Markush groups or other grouping of alternatives, those skilled in the art will recognize that the invention is also thereby described in terms of any individual member or subgroup of members of the Markush group or other group.

### Examples

### Example 1: Formulation Preparation

To a 600 gallon jacketed stainless steel kettle equipped with a primary scraper and a secondary mixer is added 396 grams of benzethonium chloride in about 602 grams of purified water at a temperature of 55° to 65°C. The benzethonium chloride container is rinsed with an addition of 602 grams of water. To the solution in the kettle is added 118.8 kg of ethanol and 99.0 kg of propylene glycol.

19.8 kg of vitamin E acetate, 200.6 kg of phospholipon 90H HYD lecithin, and 19.8 kg of diclofenac sodium is added in sequence to an auxiliary tank at 55° C to 65° C and mixed for about 15 minutes. The auxiliary tank is weighed and evaporated alcohol is replaced. The material in the auxiliary tank is passed into the manufacturing kettle at a rate of 3 to 5 kg/minute and the auxiliary tank is rinsed twice with 13.2 kg of water at 55°C to 65°C. The bulk material in the manufacturing kettle is passed through a 60 mesh screen during circulation. The circulation rate is about 95 kg to 105 kg per minute.

The vessel bulk mixture is stirred and cooled to 30° C and after cooling for about 45 minutes a sample is removed and tested for temperature and mean volume vesicle particle size. When the temperature is about 32° C and the liposome vesicle particle size is between about 2 and 10 microns agitation is stopped.

### Example 2: Clinical Study-Osteoarthritis

A clinical study was performed in horses with osteoarthritis utilizing the 1% of diclofenac formulation described in Example 1. Lameness was graded using the AAEP (American Association of Equine Practitioners) lameness scale. Pain, joint mobility and lameness were also evaluated. The clinical investigator was completely masked to study treatment. Each tube of test article was identified only by study case number. The study was conducted for five days. During the study, between about 20 to 114 mg diclofenac were applied twice daily. On average, each dose was achieved by applying a five-inch ribbon of suspension (approximately 73 mg diclofenac per dose).

The study was performed on 116 horses in eight states. The test article had a significant positive effect on lameness, pain and owner's evaluation of lameness. There was no significant effect on joint mobility in this study.

### Results

| **Parameter** | **Placebo** | **Diclofenac** | **Comments** |
|---|---|---|---|
| **Lameness** | | | |
| ΔL (S.D.) | -0.02 (1.12) | -1.48 (1.07) | p < 0.0001 |
| Lameness Improved | 17/56 (30%) | 46/60 (77%) | p< 0.0001 |
| | | | |
| **Pain** | | | |
| ΔP(S.D.) | 0.00 (0.91) | -0.48 (0.68) | p=0.0025 |
| Pain Improved | 15/56 (27%) | 23/60 (38%) | p=0.1299 |
| | | | |
| **Mobility Improved** | 9/56 (16%) | 15/60 (25%) | p=0.1694 |
| | | | |
| **Owner Improved** | 21/56 (38%) | 47/59 (80%) | p<0.0001 |

Of the 60 horses that received the test article, no adverse reactions were attributable to topical administration of diclofenac.

This study showed that the presently disclosed 1% diclofenac liposomal suspension, applied twice daily was effective in improving three of the four study parameters. Statistically, the effect was highly significant.

Pivotal target animal safety studies were conducted with treatment doses of 0.6, 1.7, 2.8 and 5.6 times the effective dose of 73 mg twice daily. There were no toxic reactions observed, based upon physical examination, CBC, serum biochemistry, gastroscopy gross necropsy and histopathology. These data support the safety of doses above 73 mg twice daily and treatment duration of 10 days.

A preparation containing 2% diclofenac processed in a similar manner did not contain phospholipid vesicles and was not effective.

### Example 3 Clinical Study - Navicular- Syndrome

Exploratory clinical studies were conducted by veterinarians in California, Colorado and Kentucky. Each veterinarian identified horses with chronic navicular syndrome. Each veterinarian applied a five-inch ribbon of 1% of diclofenac formulation described in Example 1 twice daily for 10 days. The formulation was applied to the coronary band and bulbs of the heals. Approximately 10 horses were treated. In this study, the veterinarians judged all the treated horses were improved after treatment with the diclofenac suspension. The horses retained that improvement for approximately seven (7) days after the application was discontinued.

## Claims

1. Use of a formulation comprising:
(a) 0.1% to 5% diclofenac; and
(b) 0.5% to 20% phospholipids
for the manufacture of a pharmaceutical for treating navicular syndrome in a horse comprising topically administering said formulation to the horse.

2. The use according to claim 1, wherein the formulation further comprises 0.1% to 10% vitamin E.

3. The use according to claim 1, wherein the formulation further comprises 1% to 20% alkylane glycol, and 1% to 50% (C₁-C₆) alcohol.

4. The use according to claim 1, wherein the formulation comprises:
(a) 1% diclofenac salt,
(b) 5% propylene glycol,
(c) 6% ethanol,
(d) 1% vitamin E acetate,
(e) 10% phospholipid, and
(f) 77% water.

5. The use according to claim 1, wherein the formulation comprises 2 to 10 µm liposomes.

6. The use according to claim 1, wherein the formulation is topically administered twice daily.

7. The use according to claim 1, wherein 20 mg to about 120 mg of diclofenac is applied as a single dose to the horse.

8. The use according to claim 1, wherein 70 mg to about 80 mg of diclofenac is applied as a single dose to the horse.

9. The use according to claim 6, wherein the two doses per day are applied to the horse for at least three days.

10. The use according to claim 2, wherein the vitamin E is a C₂-C₆ ester of vitamin E.

11. The use according to claim 10, wherein the vitamin E is vitamin E acetate.

12. The use according to claim 1, wherein the diclofenac is a diclofenac salt.

13. The use according to claim 3, wherein the alkylane glycol is propylene glycol.

14. The use according to claim 3, wherein the C₁-C₆ alcohol is ethanol.

## Patentansprüche

1. Verwendung einer Formulierung, die umfasst:
(a) 0.1% bis 5% Diclofenac; und
(b) 0.5% bis 20% Phospholipide
für die Herstellung eines Arzneimittels zur Behandlung eines Navikularsyndroms bei einem Pferd, die die topische Verabreichung der Formulierung an das Pferd umfasst.

2. Die Verwendung gemäß Anspruch 1, wobei die Formulierung weiter 0.1% bis 10% Vitamin E umfasst.

3. Die Verwendung gemäß Anspruch 1, wobei die Formulierung weiter 1% bis 20% Alkylanglykol und 1% bis 50% (C₁-C₆) Alkohol umfasst.

4. Die Verwendung gemäß Anspruch 1, wobei die Formulierung umfasst:
(a) 1% Diclofenacsalz
(b) 5% Propylenglykol
(c) 6% Ethanol
(d) 1% Vitamin E Acetat
(e) 10% Phospholipid, und
(f) 77% Wasser.

5. Die Verwendung gemäß Anspruch 1, wobei die Formulierung 2 bis 10 µm Liposome umfasst.

6. Die Verwendung gemäß Anspruch 1, wobei die Formulierung zweimal am Tag topisch verabreicht wird.

7. Die Verwendung gemäß Anspruch 1, wobei 20 mg bis ungefähr 120 mg Diclofenac als Einzeldosis an das Pferd verabreicht wird.

8. Die Verwendung gemäß Anspruch 1, wobei 70 mg bis ungefähr 80 mg Diclofenac als Einzeldosis an das Pferd verabreicht wird.

9. Die Verwendung gemäß Anspruch 6, wobei die beiden Dosen pro Tag dem Pferd mindestens drei Tage verabreicht werden.

10. Die Verwendung gemäß Anspruch 2, wobei das Vitamin E ein C₂-C₆ Ester von Vitamin E ist.

11. Die Verwendung gemäß Anspruch 10, wobei das Vitamin E Vitamin E Acetat ist.

12. Die Verwendung gemäß Anspruch 1, wobei das Diclofenac ein Diclofenacsalz ist.

13. Die Verwendung gemäß Anspruch 3, wobei das Alkylanglykol Propylenglykol ist.

14. Die Verwendung gemäß Anspruch 3, wobei der C₁-C₆ Alkohol Ethanol ist.

## Revendications

1. Utilisation d'une formulation qui comprend :
(a) de 0,1 % à 5 % de diclofénac ; et
(b) de 0,5 % à 20 % de phospholipides ;
pour la fabrication d'un produit pharmaceutique destiné au traitement du syndrome naviculaire chez un cheval, laquelle utilisation comprend l'administration topique de ladite formulation au cheval.

2. Utilisation selon la revendication 1, dans laquelle la formulation comprend en outre de 0,1 % à 10 % de vitamine E.

3. Utilisation selon la revendication 1, dans laquelle la formulation comprend en outre de 1 % à 20 % d'alkylane glycol ; et de 1 % à 50 % d'alcool en C₁-C₆.

4. Utilisation selon la revendication 1, dans laquelle la formulation comprend :
(a) 1 % de sel de diclofénac,
(b) 5 % de propylèneglycol,
(c) 6 % d'éthanol,
(d) 1 % d'acétate de vitamine E,
(e) 10 % de phospholipide, et
(f) 77 % d'eau.

5. Utilisation selon la revendication 1, dans laquelle la formulation comprend des liposomes de 2 à 10 µm.

6. Utilisation selon la revendication 1, dans laquelle la formulation est administrée par voie topique deux fois par jour.

7. Utilisation selon la revendication 1, dans laquelle de 20 mg à environ 120 mg de diclofénac sont appliqués sous la forme d'une dose unique au cheval.

8. Utilisation selon la revendication 1, dans laquelle de 70 mg à environ 80 mg de diclofénac sont appliqués sous la forme d'une dose unique au cheval.

9. Utilisation selon la revendication 6, dans laquelle les deux doses quotidiennes sont appliquées au cheval pendant au moins trois jours.

10. Utilisation selon la revendication 2, dans laquelle la vitamine E est un ester en C₂-C₆ de vitamine E.

11. Utilisation selon la revendication 10, dans laquelle la vitamine E est l'acétate de vitamine E.

12. Utilisation selon la revendication 1, dans laquelle le diclofénac est un sel de diclofénac.

13. Utilisation selon la revendication 3, dans laquelle l'alkylane glycol est le propylène glycol.

14. Utilisation selon la revendication 3, dans laquelle l'alcool en C₁-C₆ est l'éthanol.
